# FASCICULE DE BREVET EUROPEEN

(11) **EP 3 044 565 B1**
(45) Date de publication et mention de la délivrance du brevet: **13.04.2022**
(21) Numéro de dépôt: 14759022.8
(22) Date de dépôt: 30.07.2014
(51) Int. Cl.: G01N 1/22, G01N 1/10

(54) **BOÎTIER DANS LEQUEL EST INTEGRÉ UN DISPOSITIF D'ÉCHANTILLONNAGE DE GAZ ET STATION DE REMPLISSAGE COMPRENANT UN TEL BOÎTIER**
KASTEN, IN DENEN EINE GASPROBENENTNAHMEVORRICHTUNG INTEGRIERT IST, UND TANKSTELLE MIT SOLCH EINEM KASTEN
BOX IN WHICH IS INTEGRATED A GAS SAMPLING DEVICE AND FILLING STATION COMPRISING SUCH A BOX

(30) Priorité: 12.09.2013 FR 1358752
(43) Date de publication de la demande: 20.07.2016
(73) Titulaire: L'Air Liquide Société Anonyme pour l'Etude et l'Exploitation des Procédés Georges Claude, 75007 Paris (FR)
(72) Inventeur: CARTEAU, David, F-92800 Puteaux (FR); MAUVAIS, Patrick, F-78610 Auffargis (FR)
(74) Mandataire: Air Liquide
(86) Numéro de dépôt international: PCT/FR2014/051982
(87) Numéro de publication internationale: WO 2015/036669

(56) Documents cités:
- CN-A- 101 418 908
- FR-A1- 2 673 722
- US-A- 5 101 671
- US-A- 5 116 330
- US-A- 5 209 102
- US-A- 5 437 199
- US-A1- 2002 170 364
- US-A1- 2006 172 428
- US-A1- 2007 157 969

## Description

La présente invention concerne un boîtier dans lequel est intégré un dispositif d'échantillonnage de gaz ainsi qu'une station de remplissage de réservoirs comprenant un tel boîtier Les impuretés contenues dans l'hydrogène peuvent affecter le fonctionnement des piles à combustible à membrane échangeuse de protons (PEMFC) équipant les véhicules et autres applications mobiles. Afin d'optimiser leur performance et leur durée de vie, l'hydrogène fourni aux piles à combustibles doit répondre à des exigences de qualité strictes, publiées dans des normes internationales.

L'essentiel de l'hydrogène est généralement produit via des procédés industriels tels que le reformage à la vapeur du gaz naturel (le plus communément utilisé) ou la gazéification du charbon. L'hydrogène peut également être produit via des procédés moins classiques tels la gazéification de la biomasse ou l'électrolyse de l'eau.

Des procédés de purification permettent de générer un hydrogène de très haute pureté (supérieure à 99,9% selon les cas) pouvant néanmoins contenir des impuretés dont la nature et la concentration dépendent directement du procédé mais aussi de la source de production (gaz naturel, charbon, naphta, biomasse, eau, etc.). Certaines impuretés (monoxyde de carbone CO, sulfure d'hydrogène H₂S, ammoniac NH₃) affectent de manière irréversible, ou réversible selon le cas, le fonctionnement des piles à combustibles à membranes échangeuses de protons tandis. D'autres impuretés (dioxyde de carbone, oxygène, hydrocarbures, formaldéhyde, etc.) ont un effet moindre sur le fonctionnement des piles à combustibles.

L'ensemble des impuretés est répertorié dans la norme ISO 14687-2 qui définit la nature et la concentration des espèces devant être soumises à analyse.

Afin de vérifier la qualité de l'hydrogène par des techniques d'analyse de laboratoire, des prélèvements d'hydrogène doivent être effectués au point d'utilisation, c'est-à-dire au niveau de la sortie de gaz à haute pression (350 bar ou 700 bar par exemple) des stations de remplissage.

L'analyse des impuretés est relativement difficile pour la plupart d'entre elles. Le très faible niveau de concentration recherché (par exemple de l'ordre du ppb pour H₂S et environ 200 ppb pour CO) et la réactivité de certaines espèces (H₂S, NH₃) vis-à-vis de certain matériau exigent une technique d'échantillonnage particulièrement exigeante.

Les contraintes techniques associées à cette analyse sont nombreuses, notamment :
- les pressions et débits de gaz à analyser sont élevés,
- la nécessiter de réaliser une passivation chimique des matériaux pour limiter l'adsorption des contaminants,
- les équipements doivent présenter une haute pureté,
- le matériel utilisé doit être adapté à la haute pression,
- l'équipement doit respecter les contraintes de sécurité (règlementation zone explosive « ATEX »)...

Le prélèvement d'un échantillon d'hydrogène représentatif, sans risque d'introduction de polluant même à très basse teneur (ppb = partie par milliard), est donc capital car il doit permettre d'évaluer la qualité du carburant alimentant les réservoirs remplis.

Une norme ASTM concerne l'échantillonnage de l'hydrogène.

Le document CN101418908A1 décrit une station de remplissage de réservoirs d'hydrogène intégrant un dispositif d'échantillonnage. La station de remplissage comprend un raccord de sortie dédié à l'échantillonnage et prélevant du gaz en amont des organes de régulation de débit fourni au réservoir à remplir.

Un tel dispositif permet le prélèvement d'un échantillon de gaz en même temps qu'un remplissage mais ne garantie pas que l'hydrogène prélevé est aux conditions de remplissage d'un véhicule. De plus, ce dispositif nécessite d'utiliser des organes compatibles avec une pression relativement élevée et le cas échéant incompatibles avec l'objectif de pureté du gaz.

Ce principe d'échantillonnage nécessite également des aménagements relativement coûteux ou complexes dans l'architecture de la station de remplissage.

Les documents US 5 101 671 A et US 2002/170364 A1 décrivent des dispositifs d'échantillonnage de gaz et sont également pertinents.

Un but de la présente invention est de pallier tout ou partie des inconvénients de l'art antérieur relevés ci-dessus.

A cette fin, le boîtier dans lequel est intégré un dispositif d'échantillonnage selon l'invention est défini par le revendication 1.

Par ailleurs, des modes de réalisation de l'invention peuvent comporter l'une ou plusieurs des caractéristiques suivantes:
- la conduite d'échantillonnage comprend au moins orifice calibré situé en amont du premier détendeur de pression, c'est-à-dire entre le raccord d'entrée et le premier détendeur de pression,
- la conduite d'échantillonnage comprend un orifice calibré situé en aval du premier détendeur, c'est-à-dire entre le premier détendeur et le récipient de recueil,
- la conduite d'échantillonnage comprend un premier et un second orifices calibrés disposés en série en amont du premier détendeur, c'est-à-dire situés entre le raccord d'entrée et le premier détendeur, le premier orifice calibré ayant un orifice de dimensions supérieures aux dimensions de l'orifice du second orifice calibré,
- la conduite d'échantillonnage comprend, en aval du récipient de recueil, une vanne et un second raccord de sortie pour le prélèvement de gaz échantillon issu du récipient de recueil,
- la conduite d'échantillonnage comprend un second détendeur de pression situé entre le récipient de recueil et le second raccord de sortie,
- la conduite d'évent comprend une quatrième extrémité amont munie d'une vanne et d'un clapet anti-retour, ladite quatrième extrémité amont étant raccordée à la conduite d'échantillonnage en aval du récipient de recueil,
- le système de vannes comprend une première vanne d'isolement disposée en amont du premier détendeur,
- le système de vannes comprend une seconde vanne d'isolement disposée entre le premier détendeur et le récipient de recueil,
- le système de vannes comprend une troisième vanne d'isolement disposée en aval du récipient de recueil,
- le premier détendeur est configuré pour abaisser la pression du gaz à une valeur déterminée comprise entre 10 bar et 100 bar, par exemple entre 15 et 50bar,
- le second détendeur est configuré pour abaisser la pression du gaz à une valeur déterminée comprise entre 1,5 et 5 bar et de préférence entre 2 et 3 bar,
- le récipient de recueil comprend une surface interne désactivée chimiquement c'est-à-dire traitée selon un procédé du type notamment Sulfinert^{®} de façon à éliminer ou limiter les phénomènes d'absorption/décomposition de composés réactifs.

L'invention concerne également une station de remplissage. de réservoirs d'hydrogène comprenant un boîtier intégrant un dispositif d'échantillonnage de gaz selon l'une quelconque des caractéristiques ci-dessus ou ci-après.

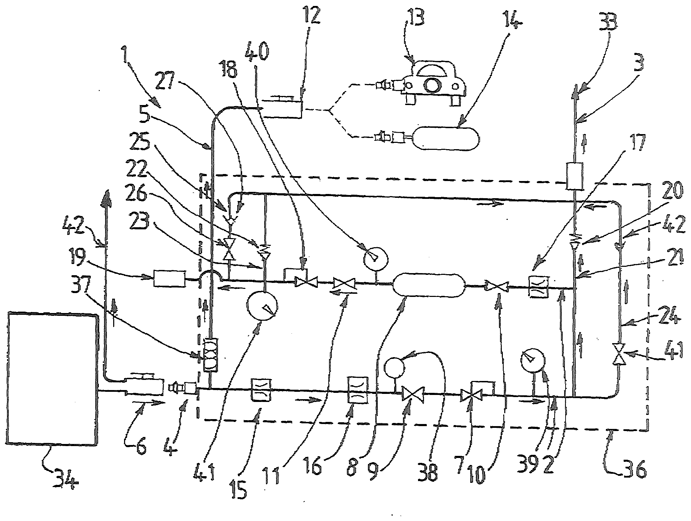

D'autres particularités et avantages apparaîtront à la lecture de la description ci-après, faite en référence à la figure unique qui représente une vue schématique et partielle illustrant un exemple de structure de dispositif d'échantillonnage coopérant avec une station de remplissage d'hydrogène.

Le dispositif d'échantillonnage de gaz pour station de remplissage de réservoirs d'hydrogène représenté à la figure comprend un circuit 2, 3, 5 fluidique comportant une première extrémité amont munie d'un raccord 4 d'entrée destiné à être raccordé de façon amovible à un raccord 6 de sortie d'une station 34 de remplissage.

C'est-à-dire que le dispositif d'échantillonnage peut être raccordé de façon amovible directement au niveau de l'extrémité aval de la station de remplissage, au niveau de la buse 6 de sortie de gaz prévue pour être raccorder à un réservoir 14 ou un véhicule 13. Alternativement, le dispositif d'échantillonnage peut être raccordé au niveau du raccord de sortie d'un compresseur ou d'un réservoir tampon d'une station 34 de remplissage.

De même, le dispositif d'échantillonnage peut être raccordé à la sortie aval d'une station de remplissage, au niveau de l'orifice prévu pour être raccordé habituellement à un flexible muni d'un pistolet ou d'une buse destinée à être raccordée aux réservoirs à remplir. C'est-à-dire que le dispositif d'échantillonnage peut être raccordé à la place d'un flexible ou en amont ou en aval d'un flexible de distribution de gaz d'une station.

Le circuit du dispositif d'échantillonnage comprend deux conduites 2, 5 respectivement une conduite 2 d'échantillonnage et un conduite 5 de remplissage reliées en parallèle au raccord 4 d'entrée.

La conduite 5 de remplissage comprend une extrémité aval munie d'un premier raccord 12 de sortie destiné à être raccordé de façon amovible à un raccord 35 d'entrée d'un réservoir 13, 14 à remplir. La conduite 5 de remplissage est de préférence dépourvue de détendeur de pression ou de vanne de régulation (ces organes sont présents en amont dans le circuit de la station 34). La station 34 peut notamment comporter un circuit d'évent 42 propre.

En revanche, la conduite 5 de remplissage comporte de préférence un dispositif 37 de sécurité anti-fuite en cas d'arrachement du flexible.

La conduite 2 d'échantillonnage comprend quant à elle un système de vannes 9, 10, 11, un premier détendeur 7 de pression et un récipient 8 de recueil d'un échantillon de gaz détendu par ledit premier détendeur 7.

La conduite 2 d'échantillonnage comprend au moins un et de préférence deux orifices calibrés disposés en série en amont du premier détendeur 7, c'est-à-dire situés entre le raccord 4 d'entrée et le premier détendeur 7. Plus précisément, la conduite 2 d'échantillonnage peut comprendre un premier 15 et un second 16 orifices calibrés, le premier orifice calibré 15 ayant un orifice de dimensions supérieures aux dimensions de l'orifice du second orifice 16 calibré.

Par exemple, le premier orifice calibré 15 a un orifice de dimension compris entre 0,5 et 2mm et de préférence égal à 1mm tandis que le second orifice 16 calibré a une dimension comprise entre 100µm et 300µm et de préférence égale à 200 µm.

Comme illustré, la conduite 2 d'échantillonnage peut comprendre une première vanne 9 d'isolement disposée en amont du premier détendeur 7, entre le second orifice 16 calibré et le premier détendeur 7.

Cette première vanne 9 permet d'isoler sélectivement la portion de circuit amont à haute pression du reste du circuit.

La conduite 2 d'échantillonnage comporte de préférence un premier manomètre 38 disposé en amont du premier détendeur 7, par exemple entre le second orifice 16 calibré et la première vanne 9 d'isolement.

Bien entendu d'autres arrangements sont possibles, par exemple la première vanne 9 d'isolement peut être disposée entre le premier orifice calibré 15 et le second orifice 16 calibré (de même pour le premier manomètre 38).

Comme illustré, la conduite 2 d'échantillonnage comprend de préférence une seconde vanne 10 d'isolement disposée entre le premier détendeur 7 et le récipient 8 de recueil.

De plus, un orifice 17 calibré supplémentaire peut être disposé en aval du premier détendeur 7, c'est-à-dire entre le premier détendeur 7 et le récipient 8 de recueil. Cet orifice calibré 17 a par exemple un orifice de dimensions comprises entre 200 et 900 µm, par exemple 500 µm.

Un second manomètre 39 peut être prévu entre le premier détendeur 7 et le récipient 8 de recueil, par exemple entre l'orifice 17 calibré et le premier détendeur 7.

Dans l'exemple illustré la conduite 2 d'échantillonnage comprend, en aval du récipient 8 de recueil, une troisième vanne 11, un second détendeur 18 (facultatif) et un second raccord 19 de sortie (de préférence auto-obturant) pour le prélèvement de gaz échantillon issu du récipient 8 de recueil.

La seconde vanne 10 d'isolement et la troisième vanne 11 d'isolement permettent d'isoler l'échantillon dans le récipient 8 respectivement au niveau de ses orifices amont et aval.

Comme représenté, un troisième manomètre 40 peut être prévu en aval du récipient 8 (entre le récipient 8 et la troisième vanne 11). Un quatrième manomètre 41 peut être prévu en aval du second détendeur 18.

Enfin, le circuit comprend une conduite 3 d'évent comprenant au moins une soupape 20, 22 sensible à la pression. La conduite 3 d'évent comporte une extrémité aval 33 d'évacuation débouchant à l'extérieur du dispositif. La conduite 3 d'évent comporte une première extrémité amont 21 raccordée à la conduite 2 d'échantillonnage, en amont du récipient 8 de recueil via une première soupape 20 sensible à la pression.

La conduite 3 d'évent comprend une seconde extrémité 23 amont raccordée à la conduite 2 d'échantillonnage, en aval du récipient 8 de recueil par exemple entre le second raccord 19 de sortie et le second détendeur 18 de pression. La seconde extrémité 23 amont peut être raccordée à la conduite 2 d'échantillonnage via une seconde vanne 22 sensible à la pression.

La conduite 3 d'évent comprend une troisième extrémité amont 24 munie d'une vanne 41 et d'un clapet 42 anti-retour et raccordée à la conduite 2 d'échantillonnage en amont du récipient 8 de recueil, par exemple entre le premier détendeur 7 et le troisième orifice 17 calibré. Cette vanne 41 permet la décompression du circuit via l'évent 3 en fin d'utilisation.

La conduite 3 d'évent peut comprendre une quatrième extrémité amont 25 munie d'une vanne 26 et d'un clapet 27 anti-retour et raccordée à la conduite 2 d'échantillonnage en aval du second détendeur 18. Cette vanne 26 permet de purger la portion aval du circuit d'échantillonnage en amont du second raccord 19 de sortie.

Les clapets anti-retour 27, 42 évitent toute pollution du circuit par l'air en cas d'ouverture des vannes 26, 41 associées.

Les soupapes de sécurité 20, 22 présentes en aval des détendeurs 7, 18 permettent d'évacuer une surpression vers l'évent 3 en cas de défaillance des détendeurs 7, 18. De préférence, l'extrémité aval 3 de l'évent forme un collecteur de longueur appropriée (3 mètres par exemple) pour évacuer le gaz inflammable loin des utilisateurs.

Le circuit d'évent permet la purge de la conduite 5 de remplissage (de préférence flexible) assurant la connexion avec le réservoir à remplir. Cette purge peut être réalisée via la seconde extrémité en ouvrant la vanne 41 correspondante.

Ceci évite une pollution du réservoir. L'utilisation du véhicule sera possible après prélèvement.

Les orifices calibrés 15 ,16 permettent ainsi de limiter le débit de gaz vers le premier détendeur 7 et le récipient 8 sans polluer le gaz destiné à être prélevé pour analyse.

De plus, en limitant le débit aval ces orifices 15, 16 calibrés permettent de diminuer la taille des soupapes 20, 22 sensibles à la pression du circuit d'évent et du collecteur 3 de ce circuit d'évent.

Le troisième orifice 17 calibré permet quant à lui de limiter le débit de remplissage du récipient 8 d'accueil. Ceci permet d'évite un échauffement trop important de celle-ci lors de son remplissage.

Le second détendeur 18 disposé en aval du récipient 8 permet une réduction de pression supplémentaire, par exemple pour distribuer le gaz prélevé dans des conditions compatibles avec les caractéristiques techniques d'un analyseur.

Les deux conduites d'échantillonnage 2 et de remplissage peuvent être mises en service simultanément lors de la connexion du raccord 4 d'entrée à la sortie 6 de gaz de la station 34 de remplissage. Ces deux lignes assurent respectivement un remplissage du réservoir 13, 14 du véhicule à haute pression et un prélèvement de gaz à basse pression dans un récipient 8.

L'échantillonnage de gaz pour l'analyse d'impuretés à basses teneurs implique l'utilisation de matériels adaptés (vannes, détendeurs, tubes, cylindre, etc.) en termes de conception technologique, de propreté, d'état et de traitement de surface.

La pression basse obtenue en aval du premier détendeur 7 (pression inférieure à 100bar) permet de trouver facilement un matériel adapté.

Dans le cas où le premier détendeur 7 est du type piloté, la rampe de pression et la pression cible pourront être contrôlées en aval. Ceci permet l'échantillonnage à des pressions et des débits plus faibles.

L'hydrogène contenu dans le récipient 8 pourra être ensuite analysé en laboratoire (par exemple mesures des impuretés pour les applications pile à combustible).

Bien que de structure simple et peu coûteuse, la solution proposée constitue un système original de prélèvement d'hydrogène à la sortie d'une station 34 de remplissage. L'échantillonnage peut être réalisé simultanément et dans des conditions identiques au remplissage d'un réservoir.

Le dispositif peut s'intercaler directement entre la station 34 de remplissage (ou un réservoir tampon) et le véhicule 13 en utilisant les connexions déjà présentes et sans nécessiter de modification du matériel.

Les manomètres 38, 39 40 (facultatifs) indiquent la pression des différentes parties du circuit.

Le dispositif d'échantillonnage est intégré dans un boîtier 36 mobile transportable manuellement, ayant un volume compris entre 0,1 et 0,3m³ une masse comprise entre 5 et 35kg.

## Revendications

1. Boîtier dans lequel est intégré un dispositif d'échantillonnage de gaz pour station de remplissage de réservoirs d'hydrogène, le boîtier étant mobile et transportable manuellement, et ayant un volume compris entre 0,1 et 0,3 m³ et une masse comprise entre 5 et 35 kg, le dispositif comprenant un circuit (2, 3, 5) comprenant une première extrémité amont munie d'un raccord (4) d'entrée et deux conduites (2, 5) respectivement d'échantillonnage (2) et de remplissage (5), la conduite (2) d'échantillonnage comprenant un système de vannes (9, 10, 11), un premier détendeur (7) de pression et un récipient (8) de recueil d'un échantillon de gaz détendu par ledit premier détendeur (7), la conduite (5) de remplissage comprenant une extrémité aval munie d'un premier raccord (12) de sortie destiné à être raccordé de façon amovible à un raccord (35) d'entrée d'un réservoir (13, 14) à remplir, où raccord d'entrée est destiné à être raccordé de façon amovible à un raccord (6) de sortie d'une station (34) de remplissage et où les deux conduites (2, 5) respectivement d'échantillonnaae et de remplissage sont reliées en parallèle au raccord (4) d'entrée, le circuit (2, 3, 5) comprenant également une conduite (3) d'évent comprenant au moins une soupape (20, 22) sensible à la pression, la conduite (3) d'évent ayant une extrémité aval (33) d'évacuation débouchant à l'extérieur du dispositf, une première extrémité amont (21) raccordée à la conduite (2) d'échantillonnage, en amont du récipient (8) de recueil, une seconde extrémité amont (23) raccordée à la conduite (2) d'échantillonnage en aval du récipient (8) de recueil, et une troisième extrémité amont (24) munie d'une vanne (41) et d'un clapet (42) anti-retour, ladite troisième extrémité amont (24) étant raccordée à la conduite (2) d'échantillonnage en amont du récipient (8) de recueil.

2. Boîtier selon la revendication 1 **caractérisé en ce que** la conduite (2) d'échantillonnage comprend au moins orifice (15, 16) calibré situé en amont du premier détendeur (7) de pression, c'est-à-dire entre le raccord (4) d'entrée et le premier détendeur (7) de pression.

3. Boîtier selon la revendication 1 ou 2 **caractérisé en ce que** la conduite (2) d'échantillonnage comprend un orifice (17) calibré situé en aval du premier détendeur (7), c'est-à-dire entre le premier détendeur (7) et le récipient (8) de recueil.

4. Boîtier selon l'une quelconque des revendications 1 à 3 **caractérisé en ce que** la conduite (2) d'échantillonnage comprend un premier (15) et un second (16) orifices calibrés disposés en série en amont du premier détendeur (7), c'est-à-dire situés entre le raccord (4) d'entrée et le premier détendeur (7), le premier orifice calibré (15) ayant un orifice de dimensions supérieures aux dimensions de l'orifice du second orifice (16) calibré.

5. Boîtier selon l'une quelconque des revendications 1 à 4 **caractérisé en ce que** la conduite (2) d'échantillonnage comprend, en aval du récipient (8) de recueil, une vanne (11) et un second raccord (19) de sortie pour le prélèvement de gaz échantillon issu du récipient (8) de recueil.

6. Boîtier selon la revendication 5, **caractérisé en ce que** la conduite (2) d'échantillonnage comprend un second détendeur (18) de pression situé entre le récipient (8) de recueil et le second raccord (19) de sortie.

7. Boîtier selon l'une quelconque des revendications 1 à 6, **caractérisé en ce que** la conduite (3) d'évent comprend une quatrième extrémité amont (25) munie d'une vanne (26) et d'un clapet (27) anti-retour, ladite quatrième extrémité amont (25) étant raccordée à la conduite (2) d'échantillonnage en aval du récipient (8) de recueil.

8. Boîtier selon l'une quelconque des revendications 1 à 7, **caractérisé en ce que** le système de vannes (9, 10, 11) comprend une première vanne (9) d'isolement disposée en amont du premier détendeur (7).

9. Boîtier selon la revendication 8, **caractérisé en ce que** le système de vannes (9, 10, 11) comprend une seconde vanne (10) d'isolement disposée entre le premier détendeur (7) et le récipient (8) de recueil.

10. Boîtier selon la revendication 9, **caractérisé en ce que** le système de vannes (9, 10, 11) comprend une troisième vanne (11) d'isolement disposée en aval du récipient (8) de recueil.

11. Station de remplissage de réservoirs d'hydrogène comprenant un boîtier d'échantillonnage de gaz selon l'une quelconque des revendications 1 à 10.

## Patentansprüche

1. Gehäuse, in welches eine Gasprobenentnahmevorrichtung für eine Station zum Befüllen von Wasserstofftanks integriert ist, wobei das Gehäuse mobil und manuell transportierbar ist und ein Volumen zwischen 0,1 und 0,3 m³ und eine Masse zwischen 5 und 35 kg aufweist, wobei die Vorrichtung einen Kreislauf (2, 3, 5) umfasst, der ein erstes stromaufwärtiges Ende mit einem Einlassanschlussstück (4) und zwei Leitungen (2, 5) jeweils zur Probenentnahme (2) und zum Befüllen (5) umfasst, wobei die Probenentnahmeleitung (2) ein System aus Ventilen (9, 10, 11), einen ersten Druckminderer (7) und einen Behälter (8) zum Auffangen einer Gasprobe umfasst, die von dem ersten Druckminderer (7) expandiert wird, wobei die Befüllleitung (5) ein stromabwärtiges Ende mit einem ersten Auslassanschlussstück (12) umfasst, das dazu bestimmt ist, abnehmbar an ein Einlassanschlussstück (35) eines zu befüllenden Tanks (13, 14) angeschlossen zu sein, wobei das Einlassanschlussstück dazu bestimmt ist, abnehmbar an ein Auslassanschlussstück (6) einer Befüllstation (34) angeschlossen zu sein und die zwei Leitungen (2, 5) zur Probenentnahme bzw. zum Befüllen parallel zum Einlassanschlussstück (4) geschaltet sind, wobei der Kreislauf (2, 3, 5) ebenfalls eine Lüftungsleitung (3) umfasst, die wenigstens ein druckempfindliches Ventil (20, 22) umfasst, wobei die Lüftungsleitung (3) ein stromabwärtiges Abführende (33), das außerhalb der Vorrichtung mündet, ein erstes stromaufwärtiges Ende (21), das stromauf des Auffangbehälters (8) an die Probenentnahmeleitung (2) angeschlossen ist, ein zweites stromaufwärtiges Ende (23), das stromab des Auffangbehälters (8) an die Probenentnahmeleitung (2) angeschlossen ist, und ein drittes stromaufwärtiges Ende (24) mit einem Ventil (41) und einer Rückschlagklappe (42) aufweist, wobei das dritte stromaufwärtige Ende (24) an die Probenentnahmeleitung (2) stromauf des Auffangbehälters (8) angeschlossen ist.

2. Gehäuse nach Anspruch 1, **dadurch gekennzeichnet, dass** die Probenentnahmeleitung (2) wenigstens eine kalibrierte Öffnung (15, 16) umfasst, die sich stromauf des ersten Druckminderers (7), das heißt zwischen dem Einlassanschlussstück (4) und dem ersten Druckminderer (7) befindet.

3. Gehäuse nach Anspruch 1 oder 2, **dadurch gekennzeichnet, dass** die Probenentnahmeleitung (2) eine kalibrierte Öffnung (17) umfasst, die sich stromab des ersten Druckminderers (7), das heißt zwischen dem ersten Druckminderer (7) und dem Auffangbehälter (8) befindet.

4. Gehäuse nach einem der Ansprüche 1 bis 3, **dadurch gekennzeichnet, dass** die Probenentnahmeleitung (2) eine erste (15) und eine zweite (16) kalibrierte Öffnung umfasst, die in Reihe stromauf des ersten Druckminderers (7) angeordnet sind, das heißt sich zwischen dem Einlassanschlussstück (4) und dem ersten Druckminderer (7) befinden, wobei die erste kalibrierte Öffnung (15) eine Öffnung mit größeren Abmessungen als die Abmessungen der zweiten kalibrierten Öffnung (16) aufweist.

5. Gehäuse nach einem der Ansprüche 1 bis 4, **dadurch gekennzeichnet, dass** die Probenentnahmeleitung (2) stromab des Auffangbehälters (8) ein Ventil (11) und ein zweites Auslassanschlussstück (19) für die Entnahme von Probengas aus dem Auffangbehälter (8) umfasst.

6. Gehäuse nach Anspruch 5, **dadurch gekennzeichnet, dass** die Probenentnahmeleitung (2) einen zweiten Druckminderer (18) umfasst, der sich zwischen dem Auffangbehälter (8) und dem zweiten Auslassanschlussstück (19) befindet.

7. Gehäuse nach einem der Ansprüche 1 bis 6, **dadurch gekennzeichnet, dass** die Lüftungsleitung (3) ein viertes stromaufwärtiges Ende (25) mit einem Ventil (26) und einer Rückschlagklappe (27) umfasst, wobei das vierte stromaufwärtige Ende (25) an die Probenentnahmeleitung (2) stromab des Auffangbehälters (8) angeschlossen ist.

8. Gehäuse nach einem der Ansprüche 1 bis 7, **dadurch gekennzeichnet, dass** das System aus Ventilen (9, 10, 11) ein erstes Absperrventil (9) umfasst, das stromauf des ersten Druckminderers (7) angeordnet ist.

9. Gehäuse nach Anspruch 8, **dadurch gekennzeichnet, dass** das System aus Ventilen (9, 10, 11) ein zweites Absperrventil (10) umfasst, das zwischen dem ersten Druckminderer (7) und dem Auffangbehälter (8) angeordnet ist.

10. Gehäuse nach Anspruch 9, **dadurch gekennzeichnet, dass** das System aus Ventilen (9, 10, 11) ein drittes Absperrventil (11) umfasst, das stromab des Auffangbehälters (8) angeordnet ist.

11. Station zum Befüllen von Wasserstofftanks umfassend ein Gasprobenentnahmegehäuse nach einem der Ansprüche 1 bis 10.

## Claims

1. Box incorporating a gas sampling device for a hydrogen tank filling station, the box being mobile and manually transportable and having a volume of between 0.1 and 0.3 m³ and a mass of between 5 and 35 kg, the device comprising a circuit (2, 3, 5) comprising a first upstream end provided with an inlet connector (4) and two pipes (2, 5), these respectively being a sampling pipe (2) and a filling pipe (5), the sampling pipe (2) comprising a system of valves (9, 10, 11), a first pressure regulator (7) and a container (8) for collecting a sample of gas expanded by said first regulator (7), the filling pipe (5) comprising a downstream end provided with a first outlet connector (12) intended to be connected removably to an inlet connector (35) of a tank (13, 14) that is to be filled, wherein the inlet connector is intended to be connected removably to an outlet connector (6) of a filling station (34), and wherein the two pipes (2, 5), these respectively being a sampling pipe and a filling pipe, are connected in parallel to the inlet connector (4), the circuit (2, 3, 5) also comprising a vent pipe (3) comprising at least one pressure-sensitive relief valve (20, 22), the vent pipe (3) having a downstream discharge end (33) opening to the outside of the device, a first upstream end (21) connected to the sampling pipe (2), upstream of the collecting container (8), a second upstream end (23) connected to the sampling pipe (2) downstream of the collecting container (8), and a third upstream end (24) provided with a valve (41) and with a nonreturn check valve (42), said third upstream end (24) being connected to the sampling pipe (2) upstream of the collecting container (8).

2. Box according to Claim 1, **characterized in that** the sampling pipe (2) comprises at least one calibrated orifice (15, 16) situated upstream of the first pressure regulator (7), namely between the inlet connector (4) and the first pressure regulator (7).

3. Box according to Claim 1 or 2, **characterized in that** the sampling pipe (2) comprises a calibrated orifice (17) situated downstream of the first regulator (7), namely between the first regulator (7) and the collecting container (8).

4. Box according to any one of Claims 1 to 3, **characterized in that** the sampling pipe (2) comprises a first (15) and a second (16) calibrated orifice which are arranged in series upstream of the first regulator (7), namely situated between the inlet connector (4) and the first regulator (7), the first calibrated orifice (15) having an orifice of dimensions greater than the dimensions of the orifice of the second calibrated orifice (16).

5. Box according to any one of Claims 1 to 4, **characterized in that** the sampling pipe (2) comprises, downstream of the collecting container (8), a valve (11) and a second outlet connector (19) for withdrawing sample gas taken from the collecting container (8).

6. Box according to Claim 5, **characterized in that** the sampling pipe (2) comprises a second pressure regulator (18) situated between the collecting container (8) and the second outlet connector (19).

7. Box according to any one of Claims 1 to 6, **characterized in that** the vent pipe (3) comprises a fourth upstream end (25) equipped with a valve (26) and with a nonreturn check valve (27), said fourth upstream end (25) being connected to the sampling pipe (2) downstream of the collecting container (8).

8. Box according to any one of Claims 1 to 7, **characterized in that** the system of valves (9, 10, 11) comprises a first isolation valve (9) arranged upstream of the first regulator (7).

9. Box according to Claim 8, **characterized in that** the system of valves (9, 10, 11) comprises a second isolation valve (10) arranged between the first regulator (7) and the collecting container (8).

10. Box according to Claim 9, **characterized in that** the system of valves (9, 10, 11) comprises a third isolation valve (11) arranged downstream of the collecting container (8).

11. Filling station for filling hydrogen tanks, comprising a gas sampling box according to any one of Claims 1 to 10.
